# EUROPÄISCHE PATENTSCHRIFT

(11) **EP 3 313 219 B1**
(45) Veröffentlichungstag und Bekanntmachung des Hinweises auf die Patenterteilung: **19.06.2019**
(21) Anmeldenummer: 16732566.1
(22) Anmeldetag: 21.06.2016
(51) Int. Cl.: A41D 13/05, A61F 5/02

(54) **VORRICHTUNG ZUM KRAFTUNTERSTÜTZTEN AUFRICHTEN EINES GENEIGTEN OBERKÖRPERS EINER PERSON**
DEVICE FOR THE FORCE-ASSISTED LIFTING OF AN INCLINED UPPER BODY OF A PERSON
DISPOSITIF DE REDRESSEMENT ASSISTÉ DU CORPS INCLINÉ D'UNE PERSONNE

(30) Priorität: 23.06.2015 DE 102015211523
(43) Veröffentlichungstag der Anmeldung: 02.05.2018
(73) Patentinhaber: Fraunhofer-Gesellschaft zur Förderung der angewandten Forschung e.V., 80686 München (DE)
(72) Erfinder: HOCHBERG, Conrad, Tokyo-to 120-0021 (JP); SCHWARZ, Oliver, 70499 Stuttgart (DE); DAUB, Urban, 70599 Stuttgart (DE)
(74) Vertreter: Rösler, Uwe
(86) Internationale Anmeldenummer: PCT/EP2016/064255
(87) Internationale Veröffentlichungsnummer: WO 2016/207135

(56) Entgegenhaltungen:
- WO-A1-2009/050302
- JP-A- 2013 132 396

## Beschreibung

### Technisches Gebiet

Die Erfindung bezieht sich auf eine Vorrichtung zum kraftunterstützen Aufrichten eines geneigten Oberkörpers einer Person, mit einem am Schulterbereich der Person abnehmbar fest anbringbaren Schulterteilt sowie einem am Hüftbereich der Person abnehmbar fest anbringbaren Hüftteil, die beide über eine Anordnung verbunden sind, die eine Vielzahl seriell angeordnete Wirbelkörper umfasst, die jeweils längs wenigstens eines mit dem Schulter- und Hüftteil jeweils endseitig verbundenen, strangartigen, elastischen oder elastisch gelagerten Zugelementes geführt sind, wobei wenigstens zwei benachbarte Wirbelkörper jeweils wenigstens einen zueinander gegenkonturiert ausgebildeten Oberflächenbereich aufweisen.

### Stand der Technik

Vorrichtungen, die vornehmlich dem Schutz des menschlichen Rückgrades bzw. der Wirbelsäule vor lastbedingten Überbeanspruchungen dienen, stellen so genannte Orthesen dar, die meist eine Anordnung aus einer Vielzahl beweglich miteinander verbundener, sogenannter Wirbelkörper aufweist, die einerseits die Oberkörperbeweglichkeit nicht allzu sehr einschränken und andererseits eine Last aufnehmende Funktion besitzen soll, um wenigstens einen die menschliche Wirbelsäule entlastenden extrakorporalen Kraftpfad zu bilden, über den äußere, auf den Oberköper einwirkende Kräfte und Momenten abgefangen und so von der Wirbelsäule fern gehalten werden können. Ein derartiger den Rücken vor unfallbedingten Krafteinwirkungen schützender Rückenprotektor, insbesondere für Motorradfahrer geht bspw. aus der Druckschrift DE 195 27 036 A1 hervor, die zumindest eine vorstehend bezeichnete Anordnung besitzt, die unter die Motorradjacke zu Zwecken einer körpernahen Fixierung getragen wird. Der bekannte Rückenprotektor verfügt über eine serielle Aneinanderreihung von jeweils plattenförmig ausgebildeten Abstützkörpern, die jeweils über elastische Verbindungselemente miteinander verbunden sind und damit eine vornüber orientierte Beugung des Oberkörpers ermöglichen, jedoch eine rückwärts gerichtete Beugung sowie auch eine beidseits orientierte Neigung des Oberkörpers zumindest stark einschränken.

Rückprotektoren der vorstehenden Art vermögen lediglich auf den Lendenbereich und unteren Schulterblattbereich beschränkte Schutzfunktionen zu entfalten, gilt es hingegen einen Schutz möglichst über die gesamte Länge der Wirbelsäule, d.h. von der Schulter bis zum Steißbein einschließlich, zu realisieren, so bedarf es eines zusätzlichen am Oberkörper lösbar fest anbringbaren Befestigungssystems zumeist in Form eines im Schulter- und Beckenbereich anbringbaren Gurt- oder Stützschalensystems. Eine derartige Rückenorthese ist in der Druckschrift DE 195 43 566 A1 beschrieben, die über ein an der Schulter sowie am Becken abstützendes Befestigungsmittel sowie einen beide Befestigungsmittel miteinander verbindende Verbindungseinheit, die eine Vielzahl von entlang der Wirbelsäule übereinander angeordnete, begrenzt gelenkig miteinander verbundene und schlagfest ausgebildete Wirbelkörper umfasst, die allesamt durch ein strangartiges, elastisches Zugelement miteinander verbunden sind. Zur Gewährleistung der Relativbeweglichkeit zwischen jeweils zwei benachbart angeordneten Wirbelkörpern ist jeweils wenigstens eine elastisch verformbare Distanzscheibe zwischen zwei Wirbelkörpern eingebracht. Der durch die Distanzscheibe axiale Abstand zweier Wirbelkörper definiert eine maximale Kippbeweglichkeit zweier Wirbelkörper.

Eine aktiv ansteuerbare Orthese zum Zwecke des Schutzes der menschlichen Wirbelsäule vor unerwartet impulsiv auftretenden Überbelastungen, wie sie bspw. im militärischen Einsatz auftreten, ist in der Druckschrift US 2014/0224849 A1 beschrieben. Grundsätzlich ähnelt der Protektoraufbau dem vorstehend beschriebenen Protektor gemäß DE 195 43 566 A1, jedoch weisen die Wirbelkörper an den jeweils sich gegenüberliegenden axialen Stirnseiten sphärisch konkav ausgebildete Ausnehmungen auf, in die jeweils ein separater Kugelkörper eingreift, der als Gelenk- sowie als Distanzkörper zwischen zwei Wirbelkörpern dient. Durch die Kugelgelenkartige Lagerung der einzelnen Wirbelkörper aufeinander wird eine weitgehend freie Beweglichkeit der Rückenprotektoranordnung erreicht. Zudem sorgen drei parallel verlaufende und die einzelnen Wirbelkörper jeweils durchsetzende Zugseile, für eine räumlich eingeschränkte Drehbeweglichkeit der Wirbelkörper relativ zueinander, wobei die jeweils zwischen einem Wirbelkörperpaar beidseits eingeschlossenen Kugeln zur Vorkehrung gegen ein seitliches Entweichen stets zugkraftbeaufschlagt von zwei Wirbelkörpern kontaktierend eingeschlossen werden. Im Falle einer sensoriell erfassbaren auf den Menschen einwirkenden mechanischen Überbelastung sieht das bekannte Protektorsystem einen impulsiv ansprechenden Aktor vor, der in Wirkverbindung mit den Zugelementen steht und diese zu straffen vermag, wodurch die Anpresskraft zwischen den Wirbelkörpern maximiert wird und Wirbelkörper gegeneinander versteifen. Auf diese Weise bildet sich ein parallel zur menschlichen Wirbelsäule verlaufender Last abführender Kraftpfad aus, wodurch die Wirbelsäule situativ geschützt wird.

Die vorstehend erläuterten Orthesen sind vornehmlich zum Wirbelsäulenschutz konzipiert, vermögen jedoch nicht oder nur in einem sehr eingeschränkten Umfang bewegungsunterstützend und muskelentlastend Bewegungsvorgänge im Oberkörperbereich zu unterstützen, so insbesondere das Aufrichten eines vornüber geneigten Oberkörpers zur erleichtern. Hierzu finden sich jedoch in den Druckschriften US 1 384 299 sowie WO 99/45863 die Aufrichtbewegung des Oberkörpers unterstützende Vorrichtungen, die einen am Oberkörper sowie am Oberschenkelbereich anbringbaren Hebelmechanismus umfassen, der ein Arbeiten in gebeugter Haltung muskulär entlastend zu unterstützen vermag. Die bekannten Vorrichtungen sind allerdings nicht alltagstauglich, zumal bspw. ein Hinsetzen der Person konstruktionsbedingt nicht möglich ist.

Aus den Druckschriften DE 297 05 124 sowie US 4 829 989 gehen gleichsam Vorrichtungen zur aktiven Unterstützung einer den Oberkörper aufrichtenden Bewegung hervor, die jeweils ein federkraftbeaufschlagtes, gelenkig miteinander verbundenes Bügelpaar vorsehen, mit einem ersten, am Brustbereich anlegbaren Bügelteil und einem zweiten am vorderen Oberschenkelbereich anlegbaren zweiten Bügelteil. Beugt sich die Person nach vorne, zu nähern sich beide Bügelteile einander an und das Federgelenkt erfährt eine rotationsbedingte mechanische Federspannung, wodurch ein auf den Oberkörper wirkendes Aufrichtmoment erzeugt wird.

Ähnliche Orthesenvorrichtungen zur aktiven Unterstützung des Aufrichtens eines nach vorn geneigten Oberkörpers einer Person sind zudem aus den Druckschriften DE 196 52 416 A1 sowie der DE 10 2004 008 509 B1 zu entnehmen.

Die Druckschrift JP 2004-358196 offenbart eine Vorrichtung zur Vermeidung von Rückenschmerzen, wie beispielsweise Hexenschuss, die mit Hilfe eines Hüftgurtes sowie eines Schultergurtes am Körper einer Person befestigt wird. Die Vorrichtung verfügt über eine Vielzahl jeweils paarweise ineinander greifende Wirbelkörper, die über gegenkonturiert ausgebildete Oberflächenbereiche verfügen, die jeweils geradzylinderförmig ausgebildet sind und auf diese Weise ausschließlich eine nach vorn sowie rückwärtig orientierte Oberkörperbeugung erlaubt. Da die einzelnen Wirbelkörper über sich rückwärtig erstreckende quaderförmige Fortsätze verfügen, ist eine aus einer aufrechten Körperhaltung nach hinten gerichtete Rückwärtsbeugung verunmöglicht. Die Fortsätze bilden somit eine mechanische Begrenzung. Durch die speziell ausgebildeten Wirbelkörper 1 sind zudem weder eine Lateralflexion, das heißt ein bidirektionales seitliches Neigen des Oberkörpers, noch eine Rotation des Oberkörpers um die Hochachse möglich.

Die Druckschrift WO 2009/050302 A1 beschreibt eine Gliederkette bestehend jeweils aus kegelartig ausgebildeten Einzelgliedern, wobei jedes Einzelglied in Art einer Flasche ausgebildet ist, die über einen sphärisch ausgebildeten Flaschenkopf sowie über einen offen ausgebildeten Flaschenboden verfügt. Der sphärisch ausgebildete Flaschenkopf eines zweiten Einzelgliedes ragt in das Innere des offen ausgebildeten Flaschenbodens und wird von diesem derart umschlossen, so dass jeweils zwei Einzelglieder untrennbar miteinander verbunden sind, jedoch eine Relativbeweglichkeit zueinander besitzen.

Die Druckschrift FR 2825265 A1 offenbart ein orthopädisches Korsett mit einer Vielzahl ineinander greifender Wirbelkörper, die jeweils einen zentralen Durchgangskanal aufweisen, durch den ein sämtliche Wirbelkörper relativ zueinander zusammenhaltendes Kabel geführt ist. Ähnliche Anordnungen sind in der DE 103 59 105 A1 und DE 10 2011 076 843 A1 erläutert, die zur Entlastung der menschlichen Wirbelsäule und in erster Linie als Schutz für die Wirbelsäule im Falle eines Sturzes oder Unfalles dienen sollen.

Die Druckschrift JP 2013 132396 A beschreibt eine Vorrichtung zum kraftunterstützen Aufrichten eines geneigten Oberkörpers einer Person, bei der eine Vielzahl jeweils quaderförmig ausgebildete und paarweise über eine Scharniergelenkverbindung miteinander verbundene Wirbelkörper vorgesehen ist, deren gegenseitige Verkippung durch ein federartiges Zugelement beeinflussbar ist.

### Darstellung der Erfindung

Der Erfindung liegt die Aufgabe zugrunde eine Vorrichtung zum kraftunterstützen Aufrichten eines geneigten Oberkörpers einer Person, mit einem am Schulterbereich der Person abnehmbar fest anbringbaren Schulterteilt sowie einem am Hüftbereich der Person abnehmbar fest anbringbaren Hüftteil, die beide über eine Anordnung verbunden sind, die eine Vielzahl seriell angeordnete Wirbelkörper umfasst, die längs wenigstens eines mit dem Schulter- und Hüftteil jeweils endseitig verbundenen, strangartigen, elastischen oder elastisch gelagerten Zugelementes geführt sind, derart weiterzubilden, so dass zum einen der Tragekomfort, die Alltagstauglichkeit und die damit verbundene Akzeptanz zur Anwendung sowie auch die mechanische Aufrichtfunktion verbessert werden sollen. Die Vorrichtung sollte somit die Bewegungsfreiheit einer Person nicht oder möglichst wenig einschränken. Die Vorrichtung soll darüber hinaus möglichst kompakt, leichtgewichtig und effizient in der unterstützenden Funktion für die Aufrichtung eines vorn über oder auch seitlich gebeugten Oberkörpers einer Person sein. Darüber hinaus soll die Vorrichtung mit möglichst einfachen und kostengünstig herzustellenden Mitteln realisierbar sein und einen robusten und störungsfreien Einsatz ermöglichen. Die Anzahl der zur Realisierung einer derartigen Vorrichtung nötigen Einzelkomponenten sollte möglichst gering sein. Auch gilt es Sorge dafür zu tragen, dass die Vorrichtung einen wartungsfreiten und möglichst verschleißarmen und daher langlebigen Einsatz ermöglicht.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

In Abkehr zu üblichen Orthesen, die dem Zwecke der kraftunterstützten Aufrichtung eines nach vornüber gebeugten Oberkörpers einer Person dienen und ein Aufrichtmoment durch eine mechanische Abstützung im Beinbereich generieren und dieses vermittels einer Drehgelenk basierten Drehmomentübertragungskinematik auf den Oberkörper übertragen, beruht die lösungsgemäße Vorrichtung auf dem prinzipiellen Aufbau eines Rückenprotektors, wie eingangs erläutert, der ein am Körper der Person abnehmbar fest anbringbares Schulter- und Hüftteil vorsieht. Schulter- und Hüftteil sind über eine Anordnung mit einer Vielzahl einzelner beweglich zueinander gelagerter Wirbelkörper verbunden, die ihrerseits über wenigstens ein strangartiges, elastisches oder elastisch gelagertes Zugelement seriell miteinander verbunden sind. Lösungsgemäß weisen wenigstens zwei seriell benachbarte Wirbelkörper längs der Anordnung jeweils wenigstens einen zueinander sphärisch bzw. kugelig, bzw. kugelförmig gegenkonturiert ausgebildeten Oberflächenbereich auf, über den jeweils die wenigstens zwei Wirbelkörper unmittelbar lose und einander selbstzentrierend unter ausschließlicher Einwirkung einer vermittels des wenigstens einen Zugelementes generierbaren, zwischen beiden Wirbelkörpern wirkenden Anpresskraft anliegen, wobei das Zugelement elastisch ausgebildet oder zumindest mit einem elastischen Element mechanisch gekoppelt ist.

In vorteilhafter Weise ist das wenigstens eine strangartig ausgebildete, elastische oder elastisch gelagerte Zugelement einerseits am Schulterteil und andererseits am Hüftteil befestigt. Das strangartig ausgebildete Zugelement besteht in vorteilhafter Weise aus einem elastischen Gummiband, Gummiseil oder allgemein aus einem Elastomerstrang-Gebilde. Gleichfalls ist es möglich das Zugelement in Form eines biegsamen aber längsstabilen Stranggutes bspw. eines Stahlseils oder eines starren Stabes, bspw. in Form eines Kunststoffstabes auszubilden, längs dessen Erstreckung, vorzugsweise an der Verbindung mit dem Schulter- sowie Hüftteil, wenigstens ein längenvariables elastisches Element, vorzugsweise in Form einer Feder oder eines Elastomerelementes, vorgesehen ist und mit diesem in Wirkverbindung steht. Stellvertretend für die vorstehenden diversen Ausbildungsformen für ein strangartig ausgebildetes, elastisches oder elastisch gelagertes Zugelement ist aus Gründen einer vereinfachten weiteren Beschreibung nur von Zugelement die Rede.

Die vorzugsweise aus einer Vielzahl einzelner Wirbelkörper bestehende Anordnung dient zum einen der gegenseitigen Beabstandung zum anderen der relativen Beweglichkeit von Schulter- und Hüftteil. Anzahl, Form und Größe der einzelnen, die Anordnung zusammensetzenden Wirbelkörper sind nach Maßgabe der Oberkörperbeweglichkeit sowie von Form und Größe des Rückenbereiches der Person zu wählen. Mit Ausnahme eines mit dem Schulterteil und eines mit dem Hüftteil jeweils fest verbundenen End-Wirbelköpers reiht sich eine Vielzahl von Wirbelkörpern in serielle Abfolge zwischen den beiden End-Wirbelkörper ein, die allesamt nur über das wenigstens eine Zugelement getragen bzw. zusammengehalten werden. Die einzelnen, bevorzugt identisch ausgebildeten Wirbelkörper kontaktieren sich jeweils paarweise, stirnseitig über ihre jeweils gegenkonturiert ausgebildeten Oberflächenbereiche. Die Wirbelkörper stellen allesamt dreidimensionale Gebilde dar, die bevorzugt aus einem leichtgewichtigen, jedoch abriebfesten Kunststoffmaterial, vorzugweise mittels generativer Herstellungstechnik gefertigt sind. Jeder Wirbelkörper verfügt über zwei sich einander gegenüberliegende Oberflächenbereiche, über die jeweils zwei in der Anordnung benachbart angeordnete Wirbelkörper in unmittelbaren Flächenkontakt gelangen. Aufgrund der bevorzugten Form-Identität aller Wirbelkörper sind die zwei Oberflächenbereiche pro Wirbelkörper, die jeweils lokal begrenzte Flächenbereiche an dem Wirbelkörper darstellen, in Form und Größe gegenkonturiert ausgebildet. Zu Zwecken einer selbstzentrierenden Wirkung des Flächenkontaktes zwischen zwei benachbarten Wirbelkörper sind die Oberflächenbereiche nicht plan, sondern konkav bzw. konvex ausgebildet.

Nimmt eine die Vorrichtung tragende Person eine aufrechte Körperhaltung ein, so befinden sich das Schulter- und Hüftteil durch die orthopädisch vorgegebene Körperhaltung weitgehend vertikal übereinander. In dieser Konstellation ist die längs des wenigstens einen Zugelementes wirkende Zugkraft, die zwischen dem Schulter-und Hüftteil wirkt, derart gewählt, dass die Wirbelkörper über ihre Oberflächenbereiche gemäß ihrer seriellen Anordnung jeweils paarweise in einem Flächenkontakt stehen. Sobald die Person eine nach vornüber gebeugte Oberkörperhaltung einnimmt, erfährt das Zugelement eine dehnungsbedingte Längung. Da die Wirbelkörper vorzugsweise lose mit dem Zugelement in Wirkverbindung stehen, wandern die Wirbelkörper in Längsrichtung zur Wirbelsäule auseinander, d. h. zwischen den Oberflächenbereichen der Wirbelkörper bildet sich ein Spalt, der mehrere mm bzw. cm betragen kann. Es liegt auf der Hand, dass durch die beugungsbedingte Längung des wenigstens einen Zugelementes die zwischen dem Schulterteil und dem Hüftteil wirkende Zugkraft mit zunehmendem Beugungswinkel gleichfalls zunimmt. Durch die Wirbelkörper, die das Zugelement einige Zentimeter vom Rücken der Person beabstanden und parallel zur Wirbelsäule führen vermag das Zugelement ein auf den Oberkörper wirkendes, entgegen der nach vorn orientierten Beugung rückstellendes Aufrichtmoment auf den Oberkörper auszuüben, wodurch eine Aufrichtbewegung der Person vermittels der längs des Zugelementes zwischengespeicherten Spannenergie signifikant unterstützt wird.

In einer bevorzugten Ausführungsform sieht die das Schulter- und Hüftteil miteinander verbindende Anordnung eine Vielzahl vorzugsweise gleichförmig ausgebildete Wirbelkörper vor, die in serieller Anordnung sich jeweils paarweise an jeweils gegenkonturiert ausgebildeten Oberflächenbereichen berühren, d. h. der Oberflächenbereich eines der sich berührenden Wirbelkörperpaare besitzt eine konkave und der Oberflächenbereich des jeweils anderen Wirbelkörpers besitzt eine konvexe Oberflächenkontur, die vorzugsweise innerhalb des jeweiligen Oberflächenbereiches flächendeckend aneinander liegen. Besonders geeignet sind Oberflächenbereiche, die jeweils axialsymmetrisch ausgebildet sind. Eine maximale relative Beweglichkeit jeweils zweier seriell aneinander grenzender Wirbelkörper zueinander bieten jeweils gegenkonturiert sphärisch ausgebildete Oberflächenbereiche, die keine oder lediglich eine minimale Bewegungseinschränkung in der Oberkörperbeweglichkeit einer Person hervorrufen. Alternativ sind auch Wirbelkörper mit jeweils gerad-zylindrisch, doppelkegelförmig oder elliptisch geformten Oberflächenbereichen denkbar. Die jeweils den einzelnen nicht sphärisch ausgebildeten Geometrien zuordenbaren Symmetrieachsen verlaufen allesamt parallel zueinander und sind orthogonal zur Längserstreckung der Wirbelsäule orientiert, um auf diese Weise ein Beugen des Oberkörpers nach vorne zu ermöglichen, jedoch ein seitliches Neigen des Oberkörpers zu verhindern bzw. signifikant einzuschränken.

Um die Relativbeweglichkeit zweier jeweils seriell benachbart angeordneter Wirbelkörper, die im Wesentlichen durch die Form der Oberflächenbereiche, über die jeweils zwei Wirbelkörper miteinander in unmittelbarem gleitenden Kontakt stehen, vorgegeben ist, nicht oder nicht zu sehr einzuschränken, empfiehlt es sich, dass das wenigstens eine sämtliche Wirbelkörper miteinander verbindende Zugelement die Oberflächenbereiche möglichst nicht durchsetzt. In vorteilhafter Weise sind sämtliche Wirbelkörper über jeweils zwei, drei oder mehr jeweils parallel zueinander verlaufende Zugelemente miteinander verbunden, die jeweils endseitig sowohl mit dem Schulter- als auch mit dem Hüftteil verbunden sind. Je nach Anzahl der vorhandenen, parallel zueinander verlaufenden Zugelemente sind die einzelnen Wirbelkörper mit entsprechenden Hohlkanälen durchsetzt, durch die die Zugelemente verlaufen und die Wirbelkörper gegenseitig stabilisieren. Hierzu sind die durch die Wirbelkörper ragenden Hohlkanäle jeweils parallel zueinander orientiert und schließen in serieller Abfolge jeweils zweier benachbarter Wirbelkörper bündig für die Durchführung der einzelnen Zugelemente einander an. Zudem sind die jeden einzelnen Wirbelkörper durchsetzenden Hohlkanäle jeweils um die Oberflächenbereiche angeordnet, ohne diese zu durchsetzen, um auf diese Weise eine möglichst uneingeschränkte Relativbeweglichkeit zwischen zwei unmittelbar benachbarten Wirbelkörpern zu ermöglichen.

In vorteilhafter Weise durchragen die Zugelemente die einzelnen Hohlkanäle der Wirbelkörper lose, so dass die Wirbelkörper in serieller Anordnung gleichsam Perlen längs einer Kette beweglich angeordnet sind. Durch Beugen der Person nach vorne erfahren die Zugelemente eine Längung, wodurch sich die Wirbelkörper frei längs der Zugelemente verteilen können.

Die Anzahl der zwischen dem Schulter- und Hüftteil vorzusehenden Wirbelkörper richtet sich nach Form und Gestalt der einzelnen Wirbelkörper sowie auch der individuellen Rückenlänge der jeweiligen Person. Vorzugsweise sind sämtliche Wirbelkörper identisch ausgebildet, wie dies aus der weiteren Erläuterung unter Bezugnahme auf illustriertes Ausführungsbeispiel zu entnehmen ist. Gleichsam können die einzelnen Wirbelkörper eine individuelle form- und größenspezifische Ausprägung besitzen, um in Längserstreckung der aus einer Vielzahl einzelner Wirbelkörper bestehenden Anordnung individuell einstellbare bzw. vorgebbare Beweglichkeit sowie aber auch Möglichkeiten der Längenveränderung zu ermöglichen. So ist in einer bevorzugten Ausführungsform wenigstens ein Wirbelkörper geteilt ausgebildet und weist darüber hinaus einen Spreizmechanismus auf, durch den die zwei Wirbelkörperteile längs des wenigstens einen, beide Wirbelkörperteile durchsetzenden Zugelementes in einen vorgebbaren Abstand überführbar sind. Für die Realisierung eines derartigen Spreizmechanismus eignet sich vorzugsweise eine Gewindestange, die beidseitig in Gegengewinde eingreift, die jeweils in den sich gegenüberliegenden Wirbelkörperteilen integriert sind. Durch entsprechende Relativdrehung der Gewindestange gegenüber beiden Wirbelkörperteilen kann ein individueller Abstand zwischen beiden Wirbelkörperteilen eingestellt werden, gleichsam dem Gewindemechanismus eines sog. Wantenspanners zur Straffung von Stahlseilzügen, die der Befestigung von Masten auf einem Segelboot dienen. Neben einer individuellen Längenanpassung dient der vorstehend erläuterte Spreizmechanismus auch einer variablen Anpassung der Zugspannung längs des Zugelementes, dessen Längung zur einer Erhöhung der zwischen dem Schulter- und Hüftteil wirkenden Zugspannung führt.

Eine alternative Möglichkeit zur Beeinflussung der längs des Zugelementes wirkenden Zugspannung bzw. Zugkraft, mit der sowohl die zwischen den einzelnen Wirbelkörpern wirkende Anpress- bzw. Kontaktkraft aber insbesondere die das Aufrichten des geneigten Oberkörpers unterstützende Rückstellkraft variabel eingestellt werden kann, stellt ein Spindelmechanismus dar, der am Hüft- und/oder am Schulterteil vorgesehen ist, um den das Zugelement zu Zwecken der einseitigen Befestigung umwickelbar ist. Der Spindelmechanismus, der vergleichbar mit einer an einem Gitarrenkopf angebrachten Spannvorrichtung für eine Gitarrenseite ausgebildet ist, kann motorisch oder manuell bedienbar ausgelegt sein. Durch ein wiederholtes Umwickeln wenigstens eines Endbereiches des Zugelementes um die Spindel des Spindelmechanismus kann die effektive Länge des Zugelementes verändert und somit die längs des Zugelementes wirkende Zugkraft vorgegeben und somit personen- und anwendungsspezifisch eingestellt werden.

Denkbar ist es überdies, dass im Falle einer elektromotorischen Aktivierung des Spindelmechanismus die Längenvariation des wenigstens einen Zugelementes situationsbedingt und automatisch vorgenommen werden kann, bspw. durch geeignet vorhandene Sensoren, die die Körperhaltung einer Person zu erfassen vermögen. In Abhängigkeit von der Körperhaltung kann so die künstlich auf den Rücken der Person einwirkende Rückstellkraft, die die Person in eine aufrechte Haltung überzuführen vermag, individuell im Wege einer Regelung oder Steuerung automatisch eingestellt werden.

Das Schulter- und Hüftteil können in an sich bekannter Weise ausgebildet werden, bspw. in Form eines an sich bekannten Gurtsystems oder eines geeignet an die Ergometrie der Person angepassten und ggf. gepolsterten Schalensystems. Beide Elemente sind vornehmlich unter Maßgabe von Bequemlichkeit und geringem Gewicht auszubilden und an die jeweilige Person anzupassen, um Druckstellen zu vermeiden und die Alltagstauglichkeit der Vorrichtung zu gewährleisten.

Das vornehmliche Ziel der lösungsgemäßen Vorrichtung dient dem kraftunterstützten Aufrichten des Oberkörpers einer Person, die ggf. zusätzlich schwere Lasten anzuheben hat. Zur Kraftunterstützung dienen die zwischen dem Schulter- und Hüftteil längs der Wirbelsäule, d.h. längs des Rückens von dem Halswirbel- bis zu dem Lendenwirbelbereich verlaufenden, vorzugsweise als Gummibänder ausgebildeten Zugelemente, die als Federn wirken und den Oberkörper kraftunterstützt aufzurichten vermögen. Die Vorrichtung dient einer biomechanischen Entlastung des Oberkörpers, wodurch die Person zudem eine biomechanisch optimalere Haltung einzunehmen vermag.

Neben dem Aspekt eines kraftunterstützten Aufrichtens des Oberkörpers kann die lösungsgemäße Vorrichtung aufgrund der Möglichkeit die Vorspannung des Zugelementes variabel einzustellen, auch als Trainings- oder Rehabilitationsgerät eingesetzt werden. Dabei kann bspw. die Rückenmuskulatur beim Durchführen eines Beugetrainings durch variables Einstellen der längs der Zugelemente wirkenden Zugkräfte gezielt trainiert werden.

Auch ist es denkbar die lösungsgemäße Vorrichtung als Teil eines Exoskeletts einzusetzen, bspw. im Rahmen eines Exoskelettroboters, der gezielt Hebeaufgaben zu übernehmen hat. Weitere Einzelheiten können in der Beschreibung im Weiteren unter Bezugnahme auf konkrete Ausführungsbeispiele entnommen werden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a-d: Schematische Darstellung der an einer Person getragenen Vorrichtung,
- Fig. 2 a, b, c: Perspektivische Zweiseitendarstellung der Vorrichtung sowie Wirbelkörperalternative
- Fig. 3a, b: Detaildarstellung eines Wirbelkörpers

### Wege zur Ausführung der Erfindung, gewerbliche Verwendbarkeit

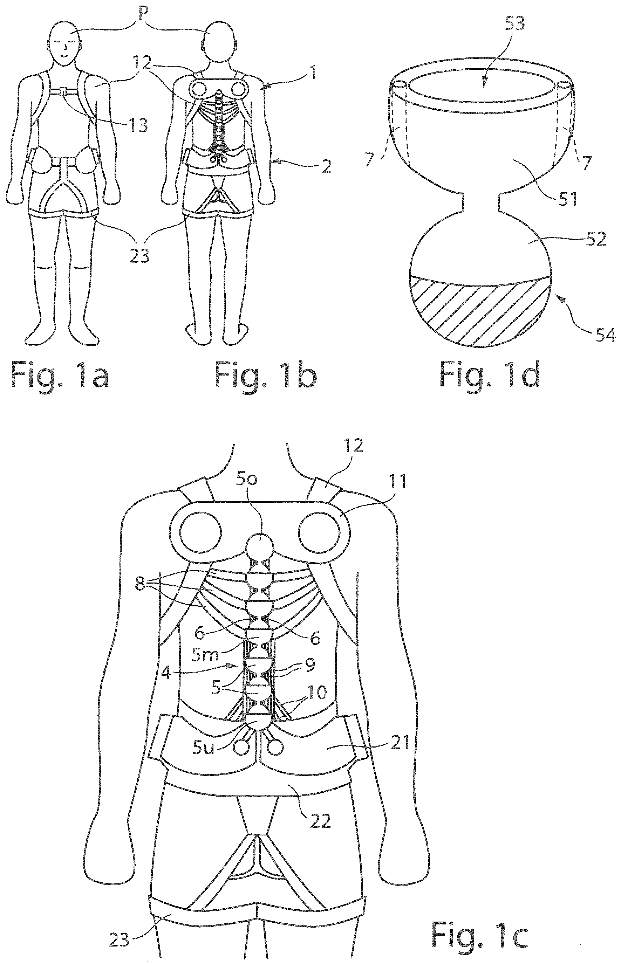

Die Figuren 1a und 1b stellen eine schematische Vorder- und Rückansicht einer Person P dar, die eine bevorzugte Ausführungsform der lösungsgemäßen Vorrichtung am Körper trägt. Figur 1c zeigt eine vergrößerte Detailansicht der Rückenansicht gemäß Figur 1b.

Die Vorrichtung zum kraftunterstützten Aufrichten eines vornüber geneigten Oberkörpers einer Person P ist abnehmbar fest am Oberkörper der Person P anbringbar und umfasst ein Schulterteil 1 sowie ein Hüftteil 2, die beide über eine Anordnung 4 miteinander verbunden sind. Die Anordnung 4 weist eine Vielzahl in serieller Abfolge angeordnete Wirbelkörper 5 auf, die mit Hilfe, vorzugsweise als Gummibänder ausgebildete Zugelemente 6 längs zwischen dem Schulterteil 1 sowie dem Hüftteil 2 feder- bzw. spannkraftbeaufschlagt gegeneinander verspannt sind. Das Schulterteil 1 sieht eine am Rücken der Person anzuordnende starre Platte 11 vor, die dem Rücken der Person P zugewandt gepolstert ist und mit einem Schultergurtsystem 12, das in Art eines Tragegurtsystems eines konventionellen Wanderrucksackes ausgebildet, mit Hilfe einer Schließe 13 eng anliegend am Oberkörper der Person lösbar fest anbringbar.

Das Hüftteil 2 besteht im gezeigten Ausführungsbeispiel aus einer ergonomisch an den Hüftbereich der Person P angepassten Halbschalenanordnung 21, die über eine entsprechende Polsterung 22 am Hüftbereich der Person passgenau anliegt. Das Hüftteil 2 ist gleichsam dem Schulterelement 1 über ein Gurtsystem 23, das in Art eines Klettergurtes ausgebildet ist, lösbar fest am Hüft- bzw. Gesäßbereich anbringbar. Das Gurtsystem 23 des Hüftteils 2 umfasst die Oberschenkel der Person, um auf dieser Weise ein nach oben orientiertes Verrutschen des Hüftteils 2 längs des Körpers zu verhindern.

Sowohl die starr ausgebildete Schulterplatte 11 des Schulterteils 1 sowie auch die starr ausgebildete Halbschalenanordnung 21 des Hüftteils 2, die vorzugsweise aus einem faserverstärkten Kunststoff oder Metall gefertigt sind, dienen zur jeweils einseitigen Befestigung der aus einer Vielzahl einzelner Wirbelkörper 5 bestehenden Anordnung 4 sowie auch zur jeweils endseitigen Befestigung der Zugelemente 6. In einer bevorzugten Weiterbildung der Vorrichtung können darüber hinaus an der Schulterplatte 11 und/oder der Halbschalenanordnung 21 weitere funktionale Komponenten modulartig montiert werden, wie beispielsweise Komponenten für den Aufbau eines komplex ausgebildeten Exoskelettes.

Die aus Figur 1c entnehmbare schematische Darstellung der aus einer Vielzahl einzelner Wirbelkörper 5 bestehenden Anordnung 4 verdeutlicht zum einen die räumlich nahe Anbringung der Anordnung 4 am Rücken der Person längs des Wirbelsäulenverlaufes. Im Falle einer aufrechten, d.h. geraden Körperhaltung sind die Wirbelkörper 5 längs einer Geraden angeordnet und bilden somit die kürzeste Verbindung zwischen der Schulterplatte 11 und der Halbschalenanordnung 21. Die jeweils sowohl mit der Schulterplatte 11 als auch mit der Halbschalenanordnung 21 verbundenen Zugelemente 6 verlaufen zur gegenseitigen Halterung und Stabilisierung aller Wirbelkörper 5 durch entsprechende, in den Wirbelkörpern 5 eingebrachte Hohlkanäle und vermögen die jeweils lose aneinandergrenzenden Wirbelkörper 5 gegeneinander zu pressen. Grundsätzlich ist es möglich lediglich ein zwischen der Schulterplatte 11 und der Halbschalenanordnung 21 verspanntes Zugelement 6 vorzusehen, aus Symmetriegründen sowie auch aus Gründen einer sicheren stabilisierten Lagerung aller lose seriell aneinandergrenzenden Wirbelkörper 5 sind zwei, drei oder mehr parallel zueinander verlaufende Zugelemente 6 vorteilhaft.

Jedes einzelne Zugelement 6 kann in Art eines Gummiseils bzw. Gummibandes ausgebildet sein, das jeweils endseitig sowohl an der Schulterplatte 11 als auch der Halbschalenanordnung 21 befestigt ist. Ebenso ist es möglich, das Zugelement 6 jeweils aus einem nicht längenelastischen Material, beispielsweise in Form eines Stahlseils oder eines biegsamen aber ansonsten längenformstabilen Kunststoffstabes auszubilden. Im letzteren Fall gilt es in Längsstreckung zu jedem Zugelement ein elastisch verformbares Element einzubringen, beispielsweise in Form einer Feder oder eines Elastomers. Die Längenelastizität jedes einzelnen zwischen dem Schulter- und Hüftteil verspannten Zugelementes ist erforderlich, zumal eine Oberkörperbeugung nach vorne eine Krümmungsbedingte Abstandsvergrößerung zwischen dem Schulter- und Hüftteil zur Folge hat. Um dieser Abstandsvergrößerung zu folgen, sind die Zugelemente längselastisch ausgebildet.

Die Wirbelkörper 5 sind vorzugsweise jeweils identisch zueinander ausgebildet und liegen jeweils paarweise in der seriellen Abfolge lose einander an. Im Beispiel gemäß Figur 1c weist ein Wirbelkörper 5 die in Figur 1d schematisiert dargestellte dreidimensionale Form auf, die einen oberen kelchförmigen Abschnitt 51 sowie einen einstückig mit diesem verbundenen kugelförmig ausgebildeten unteren Abschnitt 52 besitzt. Der kelchförmige Abschnitt 51 weist einen sphärisch konkav ausgebildeten Oberflächenbereich 53 auf, wohingegen der untere kugelförmige Abschnitt 52 einen sphärisch konvex ausgebildeten Oberflächenbereich 54 aufweist, der in Figur 1d schraffiert dargestellt ist. Bei der in Figur 1b,c illustrierten seriellen Anordnung 4 münden jeweils die sphärisch konvex ausgebildeten Oberflächenbereiche 54 eines Wirbelkörpers in den gegenkonturiert sphärisch konkav ausgebildeten Oberflächenbereich 53 des jeweils benachbart angeordneten Wirbelkörpers 5. Zur gegenseitigen Stabilisierung und Sicherung der einzelnen Wirbelkörper 5 weisen diese Hohlkanäle 7 auf, die jeweils außerhalb der Oberflächenbereiche 53, 54 verlaufen und der Durchführung der jeweils zwischen der Schulterplatte 11 und der Halbschalenanordnung 21 gespannten Zugelemente 6 dienen. Mit Ausnahme des obersten Wirbelkörpers 5o, der fest mit der Schulterplatte 11 sowie des untersten Wirbelkörpers 5u, der fest mit der Halbschalenanordnung 21 verbunden ist, sind alle übrigen Wirbelkörper 5 lose gleitend aneinander gefügt. Auch die Zugelemente 6 durchlaufen lose die jeweils in den Wirbelkörpern 5 eingebrachten Hohlkanäle 7. Dies hat zur Folge, dass sich die Wirbelkörper 5 bei einer gekrümmten Körperhaltung voneinander beabstanden. Bei einer Rückbewegung in eine aufrechte Körperhaltung sorgen die jeweils gegenkonturiert ausgebildeten Oberflächenbereiche 53, 54 zur gegenseitigen Selbstzentrierung der Wirbelkörper 5.

Bei dem in Figur 1b,c entnehmbaren Ausführungsbeispiel sind zusätzlich zu den Zugelementen 6 weitere elastische Spannmittel 8, 9 und 10 vorgesehen, die zusätzliche stabilisierende Spannkräfte in die am Rücken getragene Vorrichtung einzuleiten vermögen. So üben die vorzugsweise aus vorgeformten Gummibändern bestehenden Spannmittel 8 zwischen der Anordnung 4 und dem Schultergurtsystem 12 wirkende Zugkräfte aus, durch die eine ergonomisch korrekte und aufrecht geradlinige Oberkörperhaltung unterstützt wird. Die parallel zur seriellen Anordnung 4 verlaufenden Spannmittel 9 unterstützen die parallel zur Wirbelsäule wirkenden Zugkräfte im Lendenbereich der Person. Hierfür sind die Spannmittel 9 einseitig mit der Halbschalenanordnung 21 und andererseits mittel- oder unmittelbar am mittleren Wirbelkörper 5m angelenkt. Zur weiteren, insbesondere den Lendenbereich der Person P stabilisierenden bzw. entlastenden Krafteinleitung dienen die Spannmittel 10, die einerseits an der Halbschalenanordnung 21 und andererseits mit einem unteren Wirbelkörper 5u verbunden sind.

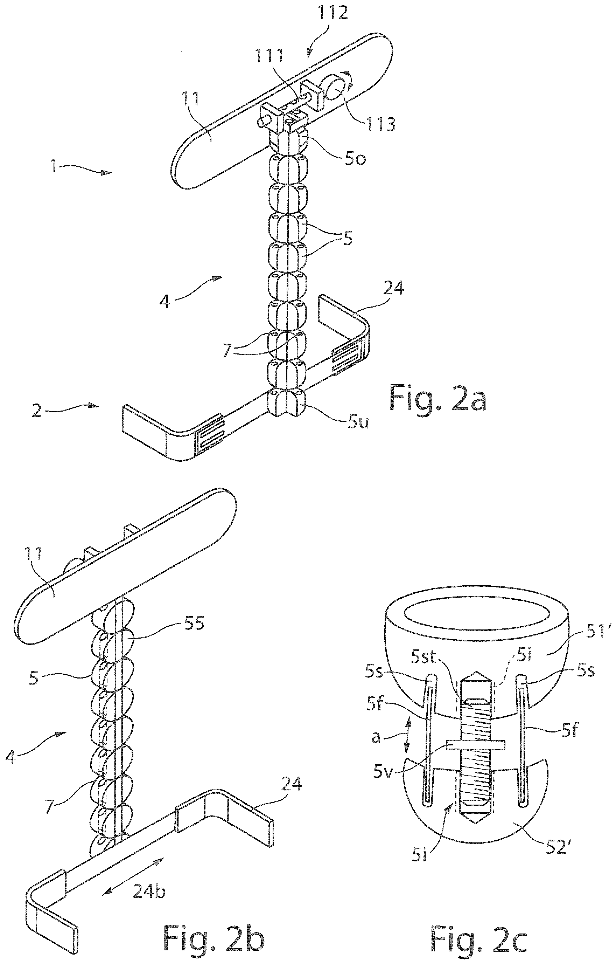

Nicht in Figur 1b,c ist eine Einheit illustriert, mit der die Zugkraft längs der jeweiligen Zugelemente 6 gezielt beeinflusst bzw. eingestellt werden kann. Eine derartige Einheit ist in Verbindung mit einer weiteren bevorzugten Ausführungsform illustriert, die in den Figuren 2a und b dargestellt ist. Die Figuren 2a und b zeigen schematisiert eine Ausführungsform zur Realisierung der Vorrichtung zum kraftunterstützenden Aufrichten eines geneigten Oberkörpers einer Person, bei der auf die Illustration des in Figur 1a und b gezeigten Gurtsystems 12 und 23 verzichtet ist. Zwischen dem als starre Schulterplatte 11 ausgebildeten Schulterteil 1 und dem Hüftteil 2 ist eine Anordnung 4 bestehend aus einer Vielzahl einzelner Wirbelkörper 5 angeordnet. Das Hüftteil 2 weist eine gabelartig ausgebildete Struktur 24 auf, deren Öffnungsbreite 24b individuell an das Hüftmaß der Person anpassbar ist, siehe Figur 2b. Gleichsam wie im vorstehend beschriebenen Ausführungsbeispiel sind der oberste Wirbelkörper 5o mit der Schulterplatte 11 und der unterste Wirbelkörper 5u mit dem Hüftteil 2 fest angeordnet. Die einzelnen Wirbelkörper 5, auf deren spezielle räumliche Ausbildung im Weiteren eingegangen wird, weisen jeweils drei Hohlkanäle 7 für die Durchführung der nicht in den Figuren 2a, b dargestellten Zugelemente auf. Die Zugelemente sind einseitig im Bereich des untersten Wirbelkörpers 5u fixiert und umschlingen mit ihrem oberen Ende eine Spindel 111 eines Spindelmechanismus 112. Der Spindelmechanismus 112 ist gleichsam dem Spannmechanismus einer Gitarrenseite ausgebildet und weist in einer bevorzugten Ausführungsform ein manuell bedienbares Griffteil 113 auf, mit dem die Spindel 111 zur Spannung oder Lockerung der vorzugsweise aus elastischen Gummibändern ausgebildeten Zugelementen beliebig oft um die Spindelachse gedreht werden kann. Ein nicht weiter dargestellter, lösbarer Rastmechanismus sorgt für die Vermeidung einer unkontrollierten rückwärtsorientierten Spindeldrehung.

Mit Hilfe des Spindelmechanismus 112 der in einer weiteren bevorzugten Ausführungsform auch mit Hilfe eines Elektromotors angetrieben werden kann, lässt sich die Zugspannung längs der Zugelemente 6 variabel einstellen.

Eine alternative Möglichkeit zur Längenvariation der Zugelemente und somit einer Einflussnahme auf die längs der einzelnen Zugelemente wirkenden Zugkraft ist in Figur 2c illustriert. In Figur 2c ist ein Wirbelkörper 5 schematisiert dargestellt, der zweigeteilt ausgebildet ist und einen oberen Teil 51' und einen unteren Teil 52' besitzt. In beiden Wirbelkörperteilen 51', 52' ist jeweils ein Innengewinde 5i eingearbeitet, das jeweils mit einem Schraubgewindestift 5st in Eingriff steht. Zusätzlich sind zwei Führungsstifte 5f vorgesehen, die in entsprechende in den Wirbelkörperhälften 51', 52' eingebrachte Sacklöcher 5s lose hineinragen und für eine stabile Längsführung beider Wirbelkörperhälften 51', 52' sorgen. Der Schraubgewindestift 5st ist über eine Stelleinheit 5v manuell oder elektromotorisch drehbar, wodurch der axiale Abstand a zwischen beiden Wirbelkörperhälften 51', 52' variabel einstellbar ist.

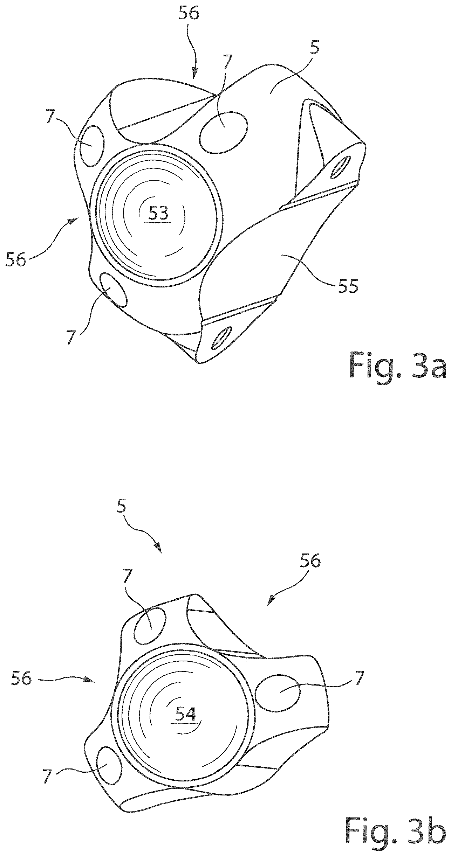

Gemäß Figuren 2a, b weisen die Wirbelkörper 5 jeweils eine modifizierte Kugelform auf, die eine dem Rücken der Person zugewandte abgeflachte Seitenfläche 55 besitzt. In den Figuren 3a, b ist eine perspektivische Ober- und Unteransicht zweier bevorzugt ausgebildeter Wirbelkörper 5 illustriert. Die dem Rücken zugewandte Seitenfläche 55 kann individuell ergonomisch an den Rücken angepasst ausgebildet sein. Zur gegenseitigen Kontaktierung der seriell längs der Anordnung 4 angeordneten Wirbelkörper 5, weist jeder Wirbelkörper 5 einen sphärisch konkav ausgebildeten Oberflächenbereich 53 (siehe Figur 3a) sowie einen diesem gegenüberliegenden sphärisch konvex ausgebildeten Oberflächenbereich 54 (siehe Figur 3b) auf. Der Wirbelkörper 5 weist darüber hinaus drei Hohlkanäle 7 auf, die jeweils außerhalb der Oberflächenbereiche 53, 54 verlaufen. Zur Zwecken der Gewichtsreduzierung sieht der Wirbelkörper 5 Materialausnehmung 56 vor, längs der optional weitere Zugelemente verlaufen können. Alternativ bietet es sich beispielsweise an, die Oberflächenbereiche 53, 54 abweichend von den in den Figuren 3a, b illustrierten sphärischen Konturen gerad-zylinderförmig, doppelkegelförmig oder elliptisch auszubilden, um auf diese Weise die Relativbeweglichkeit zwischen zwei Wirbelkörpern auf Drehbewegungen um eine Achse zu beschränken.

Im Falle der einer nach vornüber gebeugten Oberkörperhaltung liegen die einzelnen Wirbelkörper am Rücken der Person an, wobei sich jeweils benachbarte Wirbelkörper aufgrund der beugungsbedingten Streckung voneinander beabstanden. In diesem Zustand sind die Zugelemente maximal gedehnt und üben eine auf den Oberkörper nach hinten gerichtete maximale Aufrichtkraft aus. Durch die jeweils gegenkonturiert ausgebildeten Oberflächenbereiche 53, 54 zweier unmittelbar aneinandergrenzender Wirbelkörper 5 werden diese durch gegenseitige Berührung selbstzentriert und nehmen die längs der Wirbelsäule ausgerichtete Ausgangsstellung selbständig ein.

Die lösungsgemäße Vorrichtung lässt sich vollständig aus leichtgewichtigem Kunststoffmaterial fertigen und verfügt bei geeignet ergonomischer Anpassung an den Oberkörper einer Person über eine hohe Alltagstauglichkeit, zumal keinerlei Bewegungseinschränkungen, bspw. beim Sitzen, auftreten.

### Bezugszeichenliste

- 1: Schulterteil
- 11: Gurtsystem
- 12: Schultergurtsystem
- 13: Lösbare Gurtschnalle
- 2: Hüftteil
- 21: Halbschalenanordnung
- 22: Polsterung
- 23: Hüftgurt
- 24: Struktur
- 24b: Öffnungsbreite
- 4: Anordnung
- 5: Wirbelkörper
- 51,51': Oberer Wirbelkörperteil
- 52,51': Unterer Wirbelkörperteil
- 53: Konkaver Oberflächenbereich
- 54: Konvexer Oberflächenbereich
- 55: Seitenfläche
- 56: Materialausnehmung
- 5o: Oberster Wirbel körper
- 5m: Mittlerer Wirbelkörper
- 5u: Unterster Wirbel körper
- 5i: Innengewinde
- 5st: Schraubgewindestift
- 5v: Stellmechanismus
- 5f: Führungsstift
- 5s: Sackloch
- a: Axialer Abstand
- 6: Zugelement
- 7: Hohlkanal
- 8,9,10: Spannmittel
- 111: Spindel
- 112: Spindelmechanismus
- 113: Griffteil

## Patentansprüche

1. Vorrichtung zum kraftunterstützen Aufrichten eines geneigten Oberkörpers einer Person, mit einem am Schulterbereich der Person abnehmbar fest anbringbaren Schulterteil (1) sowie einem am Hüftbereich der Person abnehmbar fest anbringbaren Hüftteil (2), die beide über eine Anordnung verbunden sind, die eine Vielzahl seriell angeordnete Wirbelkörper (5) umfasst, die längs wenigstens eines mit dem Schulter- (1) und Hüftteil (2) jeweils endseitig verbundenen, strangartigen, elastischen oder elastisch gelagerten Zugelementes (6) geführt sind, wobei wenigstens zwei benachbarte Wirbelkörper (5) jeweils wenigstens einen zueinander gegenkonturiert ausgebildeten Oberflächenbereich (53, 54) aufweisen, **dadurch gekennzeichnet, dass** die wenigstens zwei Wirbelkörper (5) zur gegenseitigen Kontaktierung unmittelbar über die gegenkonturiert ausgebildeten Oberflächenbereiche (53, 54) und ausschließlich vermittels des wenigstens einen Zugelementes (6) gegenseitig elastisch kraftbeaufschlagt, lose und einander selbstzentrierend gefügt sind, und
dass die gegenkonturiert ausgebildeten Oberflächenbereiche (53, 54) sphärisch ausgebildet sind und das Zugelement (6) elastisch ausgebildet oder zumindest mit einem elastischen Element mechanisch gekoppelt ist.

2. Vorrichtung nach Anspruch 1,
**dadurch gekennzeichnet, dass** das wenigstens eine Zugelement (6) die gegenkonturiert ausgebildeten Oberflächenbereiche (53, 54) nicht durchsetzt.

3. Vorrichtung nach Anspruch 1 oder 2,
**dadurch gekennzeichnet, dass** der Oberflächenbereich (53) eines der wenigstens zwei Wirbelkörper (5) eine konkave und der Oberflächenbereich (54) des anderen der wenigstens zwei Wirbelkörper eine konvexe Oberflächenkontur aufweist.

4. Vorrichtung nach einem der Ansprüche 1 bis 3,
**dadurch gekennzeichnet, dass** an wenigstens einem Wirbelkörper (5) und/oder an dem Schulter- (1) und/oder Hüftteil (2) wenigstens eine Einheit zur Beeinflussung einer längs des wenigstens einen Zugelementes wirkenden Zugkraft angebracht ist.

5. Vorrichtung nach Anspruch 4,
**dadurch gekennzeichnet, dass** die Einheit in Form eines die effektive Länge des Zugelementes (6) variierenden Mechanismus ausgebildet ist, wobei die effektive Länge die Zugkraft des Zugelementes (6) zwischen den wenigstens zwei Wirbelkörpern (5) bestimmt.

6. Vorrichtung nach Anspruch 4 oder 5,
**dadurch gekennzeichnet, dass** die Einheit ein Spindelmechanismus (112) ist, um den das Zugelement (6) umwickelbar ist.

7. Vorrichtung nach Anspruch 4 oder 5,
**dadurch gekennzeichnet, dass** die Einheit in Art eines Spreizmechanismus ausgebildet ist, der in einem, in zwei Teile (51', 52') separierbaren Wirbelkörper integriert ist, durch den beide Teile (51', 52') längs des Zugelementes (6) in einen vorgebbaren Abstand überführbar sind.

8. Vorrichtung nach einem der Ansprüche 4 bis 7,
**dadurch gekennzeichnet, dass** die Einheit manuell bedienbar oder elektrisch aktivierbar ist.

9. Vorrichtung nach einem der Ansprüche 1 bis 8,
**dadurch gekennzeichnet, dass** die Raumform der wenigstens zwei Wirbelkörper (5) jeweils durch eine modifizierte Kugelform beschreibbar ist, die eine dem Rücken der Person zugewandte abgeflachte Seitenfläche aufweist, an der jeweils randseitig mittel- oder unmittelbar der wenigstens eine Oberflächenbereich angrenzt, an den der gegenkonturierte Oberflächenbereich des jeweils anderen Wirbelkörpers (5) zur Anlage bringbar ist,
dass jeder Wirbelkörper (5) von wenigstens einem Hohlkanal (7) durchsetzt ist, der parallel zur Seitenfläche (55) orientiert ist und den Oberflächenbereich (53, 54) nicht durchsetzt,
dass die Hohlkanäle (7) beider anliegenden Wirbelkörper (5) in einer Ausgangslage axial fluchten, und
dass das Zugelement (6) durch die Hohlkanäle (7) beider Wirbelkörper (5) geführt ist.

10. Vorrichtung nach einem der Ansprüche 1 bis 9,
**dadurch gekennzeichnet, dass** wenigstens drei, in Serie angeordnete Wirbelkörper (5) vorgesehen sind, von denen wenigstens ein Wirbelkörper (5) zwei gegenüberliegend angeordnete Oberflächenbereiche (53, 54) aufweist, die jeweils gegenkonturiert zu einem Oberflächenbereich des jeweils benachbarten Wirbelkörpers (5) ausgebildet sind, und dass die Wirbelkörper (5) durch das wenigstens eine Zugelement (6) miteinander jeweils über ihre gegenkonturiert zueinander ausgebildeten Oberflächenbereiche (53, 54) gleitend verbunden sind.

11. Vorrichtung nach einem der Ansprüche 1 bis 10,
**dadurch gekennzeichnet, dass** wenigstens zwei Zugelemente (6) vorgesehen sind, die die wenigstens zwei Wirbelkörper (5) elastisch miteinander verbinden, und dass die wenigstens zwei Zugelemente (6) die Wirbelkörper (5) parallel zueinander und gleichverteilt um die Oberflächenbereiche (53, 54) durchsetzen.

12. Vorrichtung nach einem der Ansprüche 1 bis 11,
**dadurch gekennzeichnet, dass** die Vielzahl der Wirbelkörper (5) jeweils gleichförmig ausgebildet ist.

13. Vorrichtung nach einem der Ansprüche 1 bis 12,
**dadurch gekennzeichnet, dass** das Zugelement (6) seilartig oder in Form eines biegsamen aber längsstabilen Stranggutes ausgebildet ist.

## Claims

1. Device for force-assisted straightening of an inclined upper body of a person, having a shoulder part (1) which is attachable fixedly but detachably to the person's shoulder area and a hip part (2) which is attachable fixedly but detachably to the person's hip area, both of which are connected via an assembly comprising a plurality of serially disposed vertebral bodies (5) that are guided along at least one string-like, elastic or elastically supported tensile element (6) which is connected at either end to the shoulder part (1) and the hip part (2), wherein at least two adjacent vertebral bodies (5) each have at least one surface area (53, 54) correspondingly conformed to match its counterpart,
**characterized in that**
for contacting each other, the at least two vertebral bodies (5) are joined mutually by elastic force, loosely and in self-centring manner with respect to each other directly via the correspondingly conformed surface areas (53, 54) and solely by means of the at least one tensile element (6), and
that the correspondingly conformed surface areas (53, 54) are spherical, and the tensile element (6) is constructed elastically or is at least coupled mechanically to an elastic element.

2. Device according to Claim 1,
**characterized in that** the at least one tensile element (6) does not pass through the correspondingly conformed surface areas (53, 54).

3. Device according to Claim 1 or 2,
**characterized in that** the surface area (53) of one of the at least two vertebral bodies (5) has a concave surface contour and the surface area (54) of the other of the at least two vertebral bodies has a convex surface contour.

4. Device according to any one of Claims 1 to 3,
**characterized in that** at least one unit for influencing a tensile force acting along the at least one tensile element is attached to at least one vertebral body (5) and/or to the shoulder part (1) and/or the hip part (2).

5. Device according to Claim 4,
**characterized in that** the unit is designed in the form of a mechanism that varies the effective length of the tensile element (6), wherein the effective length determines the tensile force of the tensile element (6) between the at least two vertebral bodies (5).

6. Device according to Claim 4 or 5,
**characterized in that** the unit is a spindle mechanism (112), around which the tensile element (6) can be wound.

7. Device according to Claim 4 or 5,
**characterized in that** the unit is designed in the manner of a spreader mechanism, which is integrated in a vertebral body that is separable into two parts (51', 52'), via which the two parts (51', 52') can be shifted to a predefinable distance from each other along the tensile element (6).

8. Device according to any one of Claims 4 to 7,
**characterized in that** the unit can be operated manually or activated electrically.

9. Device according to any one of Claims 1 to 8,
**characterized in that** the spatial shape of the at least two vertebral bodies (5) can be described respectively by a modified spherical shape with a flattened lateral surface closest to the person's back, each peripheral edge of which is bordered indirectly or directly by the at least one surface area, against which the correspondingly conformed surface area of the respective other vertebral body (5) can be moved up, that each vertebral body (5) is perforated by at least one hollow channel (7) which is oriented parallel to the lateral surface (55) and does not pass through the surface area (53, 54),
that the hollow channels (7) of both adjacent vertebral bodies (5) are aligned axially in an initial position, and
that the tensile element (6) is guided through the hollow channels (7) of both vertebral bodies (5).

10. Device according to one of claims 1 to 9, **characterized in that** at least three vertebral bodies (5) arranged in series are provided, of which at least one vertebral body (5) has two oppositely disposed surface areas (53, 54), each of which is conformed to correspond to a surface area of the respectively adjacent vertebral body (5), and
that the vertebral bodies (5) are connected to each other in slidable manner by the at least one tensile element (6) via their mutually correspondingly conformed surface areas (53, 54).

11. Device according to any one of Claims 1 to 10,
**characterized in that** at least two tensile elements (6) are provided which connect the at least two vertebral bodies (5) elastically with one another, and
that the at least two tensile elements (6) pass through the vertebral bodies (5) parallel to each other, and at equal distances about the surface areas (53, 54).

12. Device according to any one of Claims 1 to 11,
**characterized in that** each of the plurality of vertebral bodies (5) is constructed identically.

13. Device according to any one of Claims 1 to 12,
**characterized in that** the tensile element (6) is rope-like or is constructed in the form of a flexible, but longitudinally stable strand-like item.

## Revendications

1. Dispositif de redressement assisté par une force d'un buste incliné d'une personne, comportant une partie d'épaule (1) qui peut être montée de manière fixe amovible sur la zone d'épaule de la personne ainsi qu'une partie de hanche (2) qui peut être montée de manière fixe amovible sur la zone de hanche de la personne, qui sont toutes deux reliées par l'intermédiaire d'un dispositif, qui comprend une pluralité de corps vertébraux (5) disposés sériellement, qui sont guidés le long d'au moins un élément de traction (6), positionné élastiquement, élastique, de forme allongée, relié respectivement aux deux extrémités avec la partie d'épaule (1) et la partie de hanche (2), dans lequel au moins deux corps vertébraux voisins (5) présentent respectivement au moins une zone de surface (53,54) configurée avec un contre profil l'une par rapport à l'autre,
**caractérisé**
**en ce que** les au moins deux corps vertébraux (5) sont assemblés directement par l'intermédiaire des zones de surface (53,54) configurées avec un contre profil et exclusivement au moyen d'au moins un élément de traction (6) en étant mutuellement sollicités par une force élastique, relâchés et autocentrés l'un dans l'autre, et
**en ce que** les zones de surface configurées avec un contre profil (53,54) ont une configuration sphérique et l'élément de traction (6) est configuré élastiquement ou est couplé mécaniquement au moins avec un élément élastique.

2. Dispositif selon la revendication 1, **caractérisé en ce que** au moins un élément de traction (6) ne traverse pas les zones de surface (53,54) configurées avec un contre profil.

3. Dispositif selon la revendication 1 ou 2, **caractérisé en ce que** la zone de surface (53) d'au moins deux corps vertébraux (5) présente un contour de surface concave et la zone de surface (54) de l'autre d'au moins deux corps vertébraux présente un contour de surface convexe.

4. Dispositif selon une des revendications 1 à 3,
**caractérisé en ce que** sur au moins un corps vertébral (5) et/ou sur la partie d'épaule (1) et/ou la partie de hanche (2) au moins une unité pour influencer une force de traction agissant longitudinalement à au moins un élément de traction est montée.

5. Dispositif selon la revendication 4, **caractérisé en ce que** l'unité est configurée sous la forme d'un mécanisme variant la longueur effective de l'élément de traction (6), dans lequel la longueur effective détermine la force de traction de l'élément de traction (6) entre les au moins deux corps vertébraux (5).

6. Dispositif selon la revendication 4 ou 5, **caractérisé en ce que** l'unité est un mécanisme de broche (112), autour duquel l'élément de traction (6) peut être enroulé.

7. Dispositif selon la revendication 4 ou 5, **caractérisé en ce que** l'unité est configurée à la manière d'un mécanisme d'écartement, qui est intégré dans un corps vertébral séparable en deux parties (51',52'), par l'intermédiaire duquel les deux parties (51',52') peuvent être placées dans un écartement prescrit le long de l'élément de traction (6).

8. Dispositif selon une des revendications 4 à 7,
**caractérisé en ce que** l'unité peut être manipulée manuellement ou activée électriquement.

9. Dispositif selon une des revendications 1 à 8,
**caractérisé en ce que** la forme spatiale d'au moins deux corps vertébraux (5) peut être décrite respectivement par une forme sphérique modifiée, qui présente une surface latérale aplatie tournée vers le dos de la personne, à laquelle respectivement du côté des bords directement ou indirectement au moins une zone de surface est contiguë, sur laquelle la zone vertébrale à contre profil de l'autre corps vertébral (5) respectif peut être amenée à reposer,
**en ce que** chaque corps vertébral (5) est traversé par au moins un canal creux (7), qui est orienté parallèlement à la surface latérale (55) et ne traverse pas la zone de surface (53,54),
**en ce que** les canaux creux (7) des deux corps vertébraux contigus (5) s'alignent axialement dans une position de départ, et
**en ce que** l'élément de traction (6) est guidée à travers les canaux creux (7) des deux corps vertébraux (5).

10. Dispositif selon une des revendications 1 à 9,
**caractérisé en ce que** au moins trois corps vertébraux (5) disposés en série sont prévus, desquels au moins un corps vertébral (5) présente deux zones de surface (53,54) disposées en vis-à-vis, qui ont une configuration respectivement contre profil par rapport à une zone de surface du corps vertébral voisin respectif (5), et
**en ce que** les corps vertébraux (5) sont reliées de manière coulissante l'un à l'autre par l'intermédiaire d'au moins un élément de traction (6) via leur zone de surface (53,54) configurée avec contre profil l'une par rapport à l'autre.

11. Dispositif selon une des revendications 1 à 10,
**caractérisé en ce que** au moins deux éléments de traction (6) sont prévus, qui relient élastiquement au moins deux corps vertébraux (5) l'un à l'autre, et
**en ce que** les au moins deux éléments de traction (6) traversent les corps vertébraux (5) parallèlement l'un à l'autre et de manière uniformément répartie autour des zones de surface (53,54).

12. Dispositif selon une des revendications 1 à 11,
**caractérisé en ce que** la pluralité des corps vertébraux (5) a une configuration respectivement de forme identique.

13. Dispositif selon une des revendications 1 à 12,
**caractérisé en ce que** l'élément de traction (6) est configuré en forme de câble ou sous la forme d'un matériau allongé flexible mais stable longitudinalement.
